# EUROPÄISCHE PATENTSCHRIFT

(11) **EP 2 107 921 B1**
(45) Veröffentlichungstag und Bekanntmachung des Hinweises auf die Patenterteilung: **03.04.2013**
(21) Anmeldenummer: 07857169.2
(22) Anmeldetag: 31.12.2007
(51) Int. Cl.: A61N 2/00, A61N 2/02

(54) **VORRICHTUNG ZUM APPLIZIEREN VON GESPEICHERTEN FREQUENZINFORMATIONEN**
DEVICE FOR USING SAVED FREQUENCY INFORMATION
DISPOSITIF D'APPLICATION D'INFORMATIONS DE FRÉQUENCES MÉMORISÉES

(30) Priorität: 09.01.2007 AT 452007; 19.06.2007 AT 9502007
(43) Veröffentlichungstag der Anmeldung: 14.10.2009
(73) Patentinhaber: Lingg, Gerhard, 6900 Bregenz (AT)
(72) Erfinder: Lingg, Gerhard, 6900 Bregenz (AT)
(74) Vertreter: Puchberger, Peter
(86) Internationale Anmeldenummer: PCT/EP2007/011479
(87) Internationale Veröffentlichungsnummer: WO 2008/083847

(56) Entgegenhaltungen:
- WO-A-96/32158
- WO-A-2004/011091
- WO-A1-2006/110012
- US-A1- 2005 222 625
- US-A1- 2005 239 493
- US-A1- 2006 183 516
- US-A1- 2006 224 215

## Beschreibung

Die Erfindung betrifft eine Vorrichtung zum Applizieren von gespeicherten Frequenzinformationen auf den Körper mit einer Anzeige, einem Speicher, einer Steuerelektronik, einem D/A-Wandler, einer Spule zum Applizieren von Magnetfeldern, einem Laser zum Applizieren von Licht, sowie einer Sende- und Empfangseinrichtung zum Abfragen von Frequenzinformationen von einem Server, wobei die Vorrichtung ein Mobiltelefon (1) ist.

Es ist bekannt, dass das Applizieren von modulierten Magnetfelder und/oder moduliertem Licht auf den Körper verschiedene Auswirkungen auf dessen Funktionen haben kann. Beispielsweise können Bioinformationen als elektromagnetische Spektren aufgezeichnet und mit einer entsprechenden Vorrichtung auf den Körper übertragen werden. Je nach Substanzspektren die bei einem Stresszustand des Körpers beruhigend einwirken oder andere die bei Müdigkeit aktivierend wirken.

Die Druckschrift US 2006/0224215 A1 zeigt eine Vorrichtung zum Applizieren von gespeicherten Frequenzinformationen in Form von modulierten Magnetfeldern. Die Druckschrift WO 2006/110012 A1 zeigt eine Vorrichtung zum Applizieren von gespeicherten Frequenzinformationen, wobei die Frequenzinformationen als Laser, Vibration, Elektrizität, oder Magnetfeld auf den Körper applizierbar sind und die Vorrichtung in Form eines Bekleidungsstücks ausgeführt ist. Die Druckschrift WO 96/32158 zeigt einen therapeutischen Feldgenerator, der zum Applizieren von Frequenzinformationen auf den menschlichen Körper eingerichtet ist und einen Sensor zur Messung des Pulswertes umfasst. Die Druckschrift US 2005/02222625 A1 zeigt eine Vorrichtung zur Behandlung von Herzrhythmusstörungen durch Applizieren eines elektromagnetischen Feldes auf dem Körper. Weitere Vorrichtungen zum Applizieren von Frequenzinformationen sind aus den Druckschriften WO2004/011091 A, US 2006/183516 A1, sowie US 2005/239493 A1 bekannt.

Ein Applizieren der Frequenzinformation auf den Körper kann entweder ohne vorheriger Analyse des Momentanzustandes des Anwenders stattfinden oder aber zur genaueren Auswahl einer passenden Frequenz kann eine Zustandsanalyse mittels eines Herzraten-Variabilitäts-Herzkohärenz-(HRV)-Screenings erfolgen. Das HRV-Screening ist eine nicht invasive, über Fingersensoren abgenommene Aufnahme der autonomen nervösen Herzregulation im Sinne einer Herzraten-Variabilitäts-Herzkohärenz-Analyse. Dies gestattet Hinweise auf den allgemeinen Funktionszustand des autonomen Nervensystems. Es erlaubt Aussagen über die Stärke der sympathischen und parasympathischen Aktivität, der autonomen Balance bzw der Verschiebung der autonomen Balance in Richtung einer sympathischen oder parasympathischen Dominanz, wobei diese Aussagen wiederum mit der Stoffwechselregulation des menschlichen Organismus korrelieren und auf eine katabole bzw anabole Stoffwechsellage hindeuten, und es ermöglicht weiters einen Stresscheck durchzuführen, welcher Aussagen über physikalischen und mentalen Stress ermöglicht.

Bisher bekannt ist es Frequenzinformationen von organischen Substanzen auf einem Computer zu speichern und über daran angeschlossene Magnetspulen bzw. modulierbare Lichtquellen auf den Körper zu übertragen. Nachteilig daran ist, dass die Vorrichtungen groß sind und damit nur stationär betrieben werden können. Die Anzahl der vorhandenen Frequenzinformationen ist abhängig von der Größe des Speichers der

Vorrichtung und der Benutzer ist bisher nicht in der Lage jederzeit und überall eine entsprechende Frequenzinformation anzuwenden bzw eine Analyse seines Zustandes durchzuführen. Des weiteren sind zur Feststellung des Zustandes mittels HRV-Screening, zur Auswahl der richtigen Frequenzinformation und zum Applizieren dieser verschiedenen teiles unhandliche Vorrichtungen einige Fachkenntnis notwendig, was die Benutzerfreundlichkeit stark herabsetzt.

Es ist somit Aufgabe der Erfindung eine handliche überall zu verwendende Vorrichtung zu schaffen, welche in der Lage ist beliebig viele verschiedene Frequenzinformationen auf den Körper zu applizieren und welche weiters gegebenenfalls dazu in der Lage ist den momentanen Zustand des Anwenders mittels HRV-Screening zu analysieren.

Gelöst wird diese Aufgabe dadurch, dass die Frequenzinformationen in Form eines digitalen Dateiformats vorliegen, im Speicher des Mobiltelefons ablegbar und über den D/A-Wandler auf die Spule und den Laser übertragbar sind, wobei das Mobiltelefon zumindest zwei Sensoren zur Messung des elektrischen Potentials des Herzens sowie zumindest einen Sensor zur Messung des Pulswertes umfasst, wobei die Spule ein Magnetfeld mit einer fluktuierenden Frequenz mit einem Mittelwert von 9Hz und einer Magnetfeldstärke von 0,1 - 70µT bei einer Messentfernung von 0 Metern erzeugt, und der Laser eine Halbleiterlaserdiode ist, welche Licht mit einer fluktuierenden roten Wellenlänge mit einem Mittelwert von 650nm erzeugt, und eine Leistung von weniger als 1mW, bevorzugt etwa 0,5mW bei etwa 15mW/cm² aufweist.

Ein weiteres Merkmal der Erfindung ist es, dass das von der Spule erzeugte Magnetfeld symmetrisch quer zur Längsachse der Vorrichtung ausgerichtet sein kann.

Es können zumindest zwei, vorzugsweise drei Sensoren zur Messung des elektrischen Potentials des Herzens, sowie zumindest ein Sensor, bevorzugt in Form eines Phototransistors, zur Messung des Pulswertes vorgesehen sein.

Die zu applizierenden Frequenzinformationen liegen dabei in Form eines digitalen Dateiformats beispielsweise einer digitalen Audiodatei vor. Ein wesentlicher Vorteil der erfindungsgemäßen Vorrichtung besteht folglich darin, dass beliebig viele verschiedene Frequenzinformationen auf dem Server vorliegen können und mittels der Sende- Empfangseinrichtung jederzeit und überall abrufbar und auf den Körper mittels der Spule bzw. des Lasers applizierbar sind.

Ferner kann die Vorrichtung drei Sensoren zur Messung des elektrischen Potentials der Herzens umfassen. In einer Ausführungsform können drei Metallsensoren in Form von Elektroden aus beispielsweise Ag/AgCl beschichteten Kunststoffkörpern und ein Photosensor jeweils im Bereich der Eckpunkte der Vorrichtung vorgesehen sein, wobei pro Sensor jeweils ein Finger des Benutzers aufgelegt wird.

Die Steuerelektronik der Vorrichtung verarbeitet die von den Sensoren empfangenen Signale und berechnet eine Herzraten-Variabilitäts-Herzkohärenz-Analyse und gibt schliesslich das Ergebnis auf der Anzeige der Vorrichtung aus.

Als weiteres Merkmal der Erfindung kann die Steuerelektronik das errechnete Ergebnis mit einer im Speicher der Vorrichtung befindlichen Datenbank vergleichen und an der Anzeige der Vorrichtungen eine entsprechende Auswahl für applizierbare Frequenzinformation sowie zusätzliche weitere Informationen anzeigen, wobei die angezeigten applizierbare Frequenzinformationen mittels der Sende- und Empfangseinrichtung von einem Server herunterladbar sind.

In einer weiteren möglichen Ausführungsform sind die Sensoren, der Speicher, die Steuerelektronik, der D/A Wandler sowie die Spule und/oder das Laser der Vorrichtung in einem gemeinsamen Modul angeordnet, welches mit dem Mobiltelefon verbindbar oder in dieses einbaubar ist. Das Modul kann ferner eine eigene Stromversorgung, beispielsweise einen Akkumulator umfassen. Zur Kommunikation zwischen Modul und Mobiltelefon kann dieses mit einer Schnittstelle, wie etwa einer Bluetooth und/oder USB-Schnittstelle versehen sein.

Zusätzlich umfasst die Erfindung ein Verfahren zur Abnahme von Signalen des elektrischen Potentials des Herzens sowie des Pulswertes und zur Applizierung von gespeicherten Frequenzinformationen in Form von modulierten Magnetfeldern und/oder
*Weiter auf Seite 4 mit "moduliertem Licht auf den Körper"...* moduliertem Licht auf den Körper mittels einer oben beschriebenen Vorrichtung, wobei das Verfahren folgende Schritte umfasst:
Die Aufzeichnung der Signale über drei oder vier Sensoren an der Vorrichtung, wobei vorzugsweise ein Sensor ein Phototransistor ist und die anderen Sensoren zur Abnahme von elektrischen Signalen ausgestaltet sind. Die Erstellung einer Herzraten-Variabilitäts-Herzkohärenz-Analyse mittels der Vorrichtung. Das Vergleichen des errechneten Ergebnisses mit einer Datenbank in der Vorrichtung, wobei die Datenbank für verschiedene Ergebnisse entsprechende Informationen, wie beispielsweise Ernährungsvorschläge oder Vorschläge für mögliche applizierbare Frequenzinformationen gespeichert hat. Die Ausgabe der errechneten Ergebnisse sowie der entsprechenden Datenbankeinträge an einer Anzeige der Vorrichtung. Das optionale vom Benutzer gesteuerte Abrufen der entsprechend angezeigten Frequenzinformationen durch den Benutzer von einem Server und die Übermittlung auf die Vorrichtung mittels der Sende-Empfangseinrichtung. Die Umwandlung der übertragenen Frequenzinformationen mittels des D/A Wandlers in eine analoge Frequenz. Die Applizierung der analogen Frequenz auf den Körper mittels der Spule und/oder des Lasers der Vorrichtung sowie optional eine erneute Aufzeichnung und Erstellung einer Herzraten-Variabilitäts-Herzkohärenz-Analyse zur Überprüfung der Zustandsänderung des Benutzers.

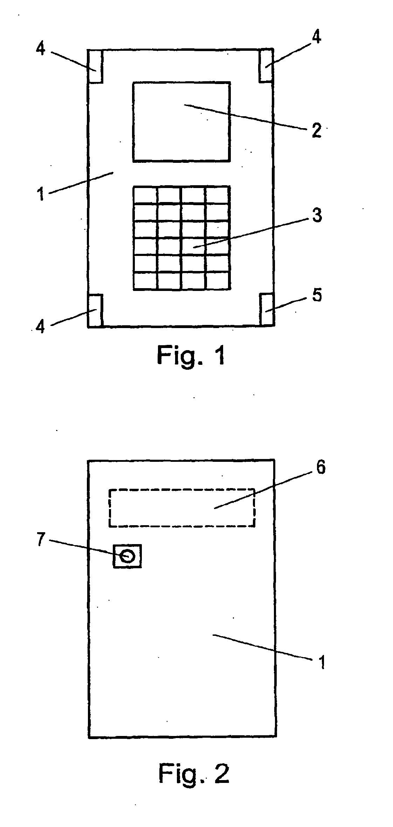

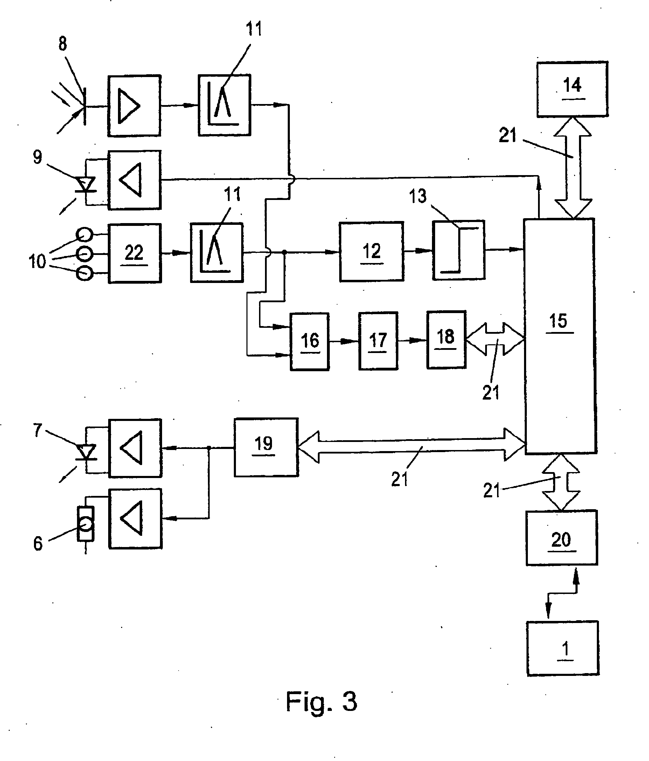

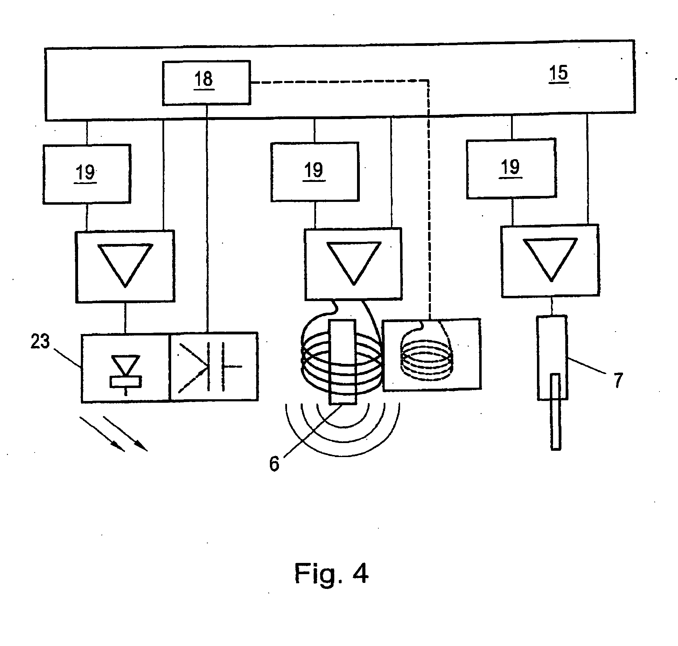

Im Folgenden wird die Erfindung anhand einiger Figuren sowie der dazugehörigen Beschreibung detaillierter erklärt, wobei Fig. 1 eine schematische Vorderansicht einer erfindungsgemäßen Vorrichtung in Form eines Mobiltelefons ist. Fig. 2 ist eine Rückansicht der erfindungsgemäßen Vorrichtung aus Fig. 1. Fig. 3 zeigt ein Blockschaltbild einer möglichen Ausführungsform der erfindungsgemäßen Vorrichtung. Fig. 4 zeigt ein Blockschaltbild einer Schaltvariante zur Ansteuerung der Aktoren.

Das in Fig. 1 schematisch dargestellte Mobiltelefon 1 mit einer Anzeige 2 und einer Tastatur 3 weist an seinen Ecken jeweils einen Sensor zur Auflage eines Fingers des Benutzers auf. Dabei sind drei Metallsensoren 4 zur Aufzeichnung eines EKG's sowie ein Photosensor 5 zur Erkennung der Pulswelle vorgesehen. Der Benutzer kann mit beiden Händen das Mobiltelefon mit jeweils einem Finger an den Eckpunkten während der Signalerfassung halten.

Fig. 2 zeigt das Mobiltelefon aus Fig. 1 aus einer Rückenansicht. Strichliert dargestellt ist die im Gehäuse befindliche von außen nicht sichtbare Spule 6, die zum Applizieren des modulierten Magnetfeldes dient. Ferner befindet sich auf der Rückseite des Mobiltelefons in dieser Ausführungsform ein Laser 7, der zum Applizieren des modulierten Lichts auf den Körper dient. Der Laser 7 kann selbstverständlich auch an anderer geeigneter Stelle angeordnet sein. Nachdem über die vier Sensoren 4,5 und nachfolgender Berechnung der Herzraten-Variabilität der Zustand des Benutzers festgestellt und eine entsprechende Frequenzinformation vorgeschlagen und von einem Server heruntergeladen wurde, kann das Mobiltelefon 1 mit seiner Rückseite auf die Haut aufgelegt werden und die Frequenzinformation als moduliertes Magnetfeld bzw moduliertes Licht auf den Körper appliziert werden.

Anhand von dem in Fig. 3 gezeigten Blockschaltbild wird nun die Funktionsweise der Vorrichtung genauer beschrieben.

Die Grundfunktionen der gezeigten Ausführungsform sowie der Ablauf einer Anwendung sind wie folgt:
- Die Analyse des Zustandes des Benutzers auf der Basis einer HRV-Analyse und einer Pulswellenkorrelation
- Vorschläge für Frequenzinformationen sowie weitere Ratschläge zur Veränderung des Zustandes des Benutzers abgerufen aus der Datenbank der Vorrichtung
- Herunterladen einer adäquaten Frequenzinformation über die Sende- und Empfangseinrichtung
- Applikation der Frequenzinformation auf den Benutzer über Magnetfeld und moduliertes Laserlicht
- Überprüfung der Zustandsveränderung durch nochmalige HRV-Analyse und Pulswellenkorrelation

Analyse des Zustandes des Benutzers:
Es wird ein 1-Kanal-EKG aufgezeichnet, das über drei Finger-Elektroden 10 abgenommen wird. Ein hochimpedanter Differenzverstärker 22 mit ausreichender Gleichtaktunterdrückung verstärkt das Quell-Signal mit einer Amplitude von etwa 1-2 mV auf ca. 500 mV und trennt eventuelle durch elektrochemische Elementbildung zwischen Haut und Elektrodenfläche erzeugte DC-Anteile ab. Ein nachgeschaltetes Filter 11 filtert 50 Hz-Brumm aus. Das so konditionierte Signal geht über den Multiplexer 16 und ein Sample&Hold 17 auf einen ADC 18. Dieser sampelt das EKG-Signal mit einer Abtastrate von 2 ms = 500 Hz. Die Steuerung der AD-Wandlung erfolgt durch einen zentralen RISC-Prozessor 15 der auch den Datentransfer vom ADC 18 zum Puffer-RAM 14 übernimmt. Die dort gespeicherten Daten werden auf Anfrage des Mobiltelefons 1 über die Bluetooth-Schnittstelle 20 übertragen. Parallel zu dieser EKG-Erfassung wird über eine spezielle auf reiner Analogtechnik basierende R-Zacken-Detektion 12 mit nachgeschaltetem Trigger 13 und anschliessende Reziprokwertbildung im Prozessor 15 ein direkter RR-Wert errechnet und ebenfalls im RAM 14 abgelegt. Auch diese Werte werden auf Anforderung des Mobiltelefons 1 übertragen. Die Messgenauigkeit der Sensoren beträgt +/- 6%.

Da für die Applikation ohnehin Prozessor 15 erforderlich ist, werden Signal-Vorverarbeitung und Filterung in Software implementiert. Dadurch ergibt sich neben der Ersparnis verhältnismäßig teurer, externer analoger Hardware der Vorteil der Adaptierbarkeit, beispielsweise auf 50 Hz /60 Hz Netzbrumm-Filterung sowie eine einfache Wartbarkeit. Damit können auch spätere Verbesserungen via Firmware-Update durch den Anwender eingespielt werden. Da über die Schnittstelle zum Mobilfunkgerät (Bluetooth) die Messdaten nicht schnell genug weitergeleitet werden können, ist das Puffern des Messdatenstroms in einem Prozessor beigefügten, externen RAM 14 erforderlich. Eventuell kann als RAM auch ein langsamerer Flash-Speicher verwendet werden, der typischerweise weniger Energie benötigt.

Im Applikations-Betrieb sollen von der Vorrichtung Frequenzinformationen über verschiedene Aktoren, optische und/oder magnetische, an den Nutzer abgegeben werden. Dabei kommt wiederum der bereits zur Zwischenspeicherung der Messdaten verwendete RAM zum Einsatz, denn die Signal-Daten müssen von der Schnittstelle zum Mobiltelefon übernommen und gepuffert werden, ehe sie als Datenstrom über den vorangeschalteten DAC 19 zu den Aktoren 6, 7 übertragen werden können. Es ist auch ein Reglerbetrieb für den Nutzer implementierbar, in dem eine "Echtzeit-Bio-Feedback-Regelschleife" zur zeitgleichen Messung von EKG- sowie Pulsdaten und der Applikation von Frequenzinformationen aufgebaut werden kann.

Als Elektrodenmaterial wird ein Ag/AgCl beschichteter Kunststoffkörper vorgeschlagen. Dieses Material weist einen optimalen Wert der elektrochemischen Spannung auf wodurch ein rascher Abbau erzeugter DC-Werte möglich ist. Eine Alternative dazu wären edelmetallbeschichtete, beispielsweise AU-beschichtete Elektroden.

Über eine IR-Lichtschranke bestehend aus einer IR-LED 9 und einem Phototransistor 8 wird die Pulswelle an einer Fingerkuppe erfasst. Das aufgenommen Signal wird gefiltert und gelangt über den zweiten Kanal des Multiplexers 16 und das S&H 17 ebenfalls auf den ADC 18. Durch alternierende AD-Wandlung zwischen EKG und PW ist eine zeitliche Korrelation der beiden Größen (im Rahmen der Samplingrate) gegeben.

Zur Minimierung des Stromverbrauchs kann die IR-LED 9 erst dann eingeschaltet werden, wenn eine R-Zacke erkannt wurde und wieder abgeschaltet werden_{,} wenn eine Pulswelle erkannt wurde.

Aus den vorliegenden Frequenzanteilen lassen sich durch hochgenaue Integration spezieller Frequenzbereiche sowohl die Stärke der sympathischen und parasympathischen Aktivität als auch das Herzrythmuskohärenzverhältnis bestimmen.

Die beiden Äste des autonomen Nervensystems, Sympathikus und Parasympathikus, arbeiten unabhängig voneinander und in der Regel antagonistisch. Der Sympathikus aktiviert den Körper, trimmt ihn auf Leistungsbereitschaft und ist bei Überaktivierung stressauslösend. Der Parasympathikus wirkt demgegenüber dämpfend und entspannend auf den Organismus, fördert die Regeneration und schützt vor Überaktivierung durch Stress.

Die relative Stärke der sympathischen und parasympathischen Aktivierung kann bei jedem Menschen unterschiedlich sein. Die beiden Teilsysteme sollten sich normalerweise in einem Balancezustand befinden. In vielen Fällen besteht jedoch eine Dominanz des einen oder anderen Systems. Eine Aussage über den Zustand der autonomen Balance ist insbesondere durch das Verhältnis sympathische zu parasympathische Aktivierung möglich.

Eine Balanceverschiebung in Richtung einer sympathischen Dominanz wirkt katabolisch und bewirkt die Energiebereitstellung zur Steigerung der Leistungsfähigkeit durch verschiedene metabolische Maßnahmen wie Blockierung der Energiespeicherung und Auflösung bestehender Energiespeicher, wie zB Eiweißabbau, Anstieg des Blutzuckerspiegels, Anstieg der freien Fettsäuren. Darüber hinaus forciert sie den Energietransport durch hämatologische (Reduktion des Plasmavolumens, Erhöhung der Hämokonzentration) und kardiovaskuläre (Blutdruckanstieg, Kontraktilitätssteigerung des Herzens, Verkürzung der Reizleitungsdauer im Herzen) Maßnahmen und blockiert irrelevante oder nicht hilfreiche Körperprozesse, wie zB Verdauungsprozesse, reproduktive Prozesse, Wachstumsprozesse und Aufbaustoffwechsel, Entzündungsreaktionen, Schmerzsensibilität und Immunreaktivität. Eine Balanceverschiebung in Richtung einer parasympathischen Dominanz wirkt in entgegengesetzter Richtung. Sie wirkt anabolisch und fördert Aufbaustoffwechsel, Entspannung, Regeneration, Stressabbau und schützt vor stressbedingten Krankheiten des Herzens und anderer Körperorgane.

Das ermittelte Ergebnis das aus den aufgezeichneten Signalen errechnet wurde wird mit einer Datenbank, welche im Speicher der Vorrichtung abgelegt ist verglichen und es können folgende Informationen an den Benutzer entsprechend dem Messergebnis ausgegeben werden:
- Die Situation der Stoffwechselregulation, ob katabol oder anabol sowie entsprechende Ernährungsvorschläge
- Die autonome Balance des autonomen Nervensystems
- Ein Stresscheck mit einem Download Vorschlag für eine geeignete zu applizierende Frequenzinformation
- Das Ergebnis des Feedback Coachings nach der Applikation einer Frequenzinformation
- Weitere Download Vorschläge für Standardanwendungen
- Applikation der Frequenzinformation:
   Die anzuwendende Frequenzinformation wurde via Download von einem Server, beispielsweise uber einen Link des Mobilfunkbetreibers in das RAM des Mobiltelefons geladen. Von dort wird es durch den Prozessor 15 gelesen über den 16-Bit-DAC 19 auf eine Magnetspule 6 in Kombination mit einem starken statischen Magnetfeld appliziert. Der Kern dieser Spule 6 wird nach dem AMS-Verfahren struktur-modifiziert sein. Diese Spule erzeugt Feldstärken von ca. 70 µT und wirkt auf den Benutzer ein. Mit demselben Frequenzinformations-Signal wird die Helligkeit einer Kleinleistungs-Halbleiterlaser-Diode 7 mit einer Leistung von weniger als 1 mW moduliert. Auch dieses Laserlicht wirkt direkt auf den Benutzer ein. Die Signale zwischen dem Prozessor 15, dem RAM 14, dem ADC 18, dem 16-Bit DAC 19 sowie der Bluetooth-Schnittstelle 20 werden über einen SPI-Bus 21 übertragen. Die Frequenzinformation kann weiters über eine Infrarot Quelle 23 appliziert werden.

Die Fig. 4 zeigt eine Schaltvariante für maximale Flexibilität bei der Ansteuerung der, die Frequenzinformation auf den Nutzer applizierenden, Aktoren. Jeder Aktor 6, 7, 23 hat seine eigene Digital-Analog-Umsetzung 19 und seine eigene Vorverstärkerstufe.

Von dem Prozessor sind folgende Aufgaben, im Extremfall alle "gleichzeitig", zu erledigen:
- Abfragen der Schnittstelle zum Mobiltelefon
- Steuerung der EKG-Signal-Fensterung
- Abtasten der EKG- und Puls-Signale
- Vorverarbeiten und Filtern der EKG- und Puls-Signale
- Zwischenspeichern der EKG- und Puls-Daten im RAM
- Bereitstellen des EKG-/Puls-Datenstroms für die Übertragung über die Schnittstelle (Bluetooth/USB)
- Wiedergeben der vom RAM gepufferten Frequenzinformationen über die Kanäle zu den Aktoren

Dafür ist die Umsetzung eines aufwändigen Interrupt-Handlings erforderlich. Bei gleichzeitigem Ablaufen so vieler Teil-Prozesse stellt das Erreichen eines optimalen Timings einen besonderen Entwicklungsaufwand dar.

Die Wahl von Bluetooth als Kommunikationsschnittstelle zwischen dem Modul und dem Mobiltelefon ist sicherlich vorteilhaft, zumal hiermit das Adaptieren verschiedener Bus-Systeme auf variierende "Träger-Hardware" geschickt umgangen werden kann. Somit kann die Verdrahtung zwischen Mobiltelefon-Einheit und Modul auf die Energieversorgung reduziert werden. Diese lässt sich zB mit lediglich zwei korrosionsgeschützten Kontakten realisieren. Wahlweise kann die Stromversorgung des Moduls auch separat erfolgen, beispielsweise mittels eigenem Akku. Dadurch wird ein 1-Platinen-Design angestrebt, um die Kosten für die Serienfertigung möglichst gering zu halten. Auf dieser Leiterplatte sind sämtliche Baugruppen des Moduls untergebracht inklusive der Sensorik und Aktorik. Das Modul kann somit kostengünstig in eine modifizierte Rückwand des Mobiltelefons eingelegt, -geschraubt oder eingeclipst werden. Für den Einbau entfällt somit jegliches Verdrahten.

Am Server liegen zB 3000 verschiedene Spektren digital gespeichert vor. Bevorzugt liegen diese Frequenzinformationsspektren als digitale Audiodatei, beispielsweise als Wavesound-Datei vor.

Substanzspektren werden mittels Einstellen in eine Eingangsspule, an die ein Filter und ein Verstärker mit einer Verstärkung von 10⁶ angeschlossen sind, für etwa 4 Min aufgenommen. Von den Substanzen abgestrahlte elektromagnetische Wellen mit Frequenzen im Bereich von 20 Hz bis 100kHz werden über die Nyquist-Frequenz digitalisiert, in einem RAM gepuffert und in einem Multiplexvorgang auf CD übertragen. Das rauschunterdrückte, gefilterte Signal wird um 10⁶ auf das ursprüngliche analoge Niveau zurückgefahren. Dieses Signal wird auf einen Server geladen und kann von dort auf die Vorrichtung der Erfindung heruntergeladen werden.

Beim Abspielvorgang der Wavesound-Datei wird bevorzugt der Bereich von 20 Hz bis 18kHz berücksichtigt und induktiv über eine Spule (Magnetfeld) mit einer fluktuierenden Frequenz mit einem Mittelwert von ca. 9 Hz mittels kohärentem Licht (Laser) appliziert. Die Oberwellen reichen bis in den Megaherzbereich, zusätzlich werden sämtliche Frequenzen des Geomagnetfeldes erzeugt und übertragen. Die Ausrichtung des Magnetfeldes ist symmetrisch quer zur langen Mittelachse der Vorrichtung. Die Genauigkeit der Festfrequenzen liegt bei +/- 0,2%. Die Magnetfeldstärke ist je nach Einstellen bevorzugt von 0,17-3,8 µTesla bei einer Messentfernung von 0 m. Es kann eine automatische Abschaltung nach 30 sec - 3 min für alle Frequenzen mit einer Genauigkeit von ca. +/- 1% erfolgen.

Der Laser arbeitet mit einer fluktuierenden roten Wellenlänge (Mittelwert 650 nm). Die Leistung beträgt ca. 0,5 mW, bei ca. 15 mW/cm² im Dauerbetrieb mit gleicher Dauer wie beim Magnetfeld. Es erfolgt keine Taktung.

## Patentansprüche

1. Vorrichtung zum Applizieren von gespeicherten Frequenzinformationen auf den Körper mit einer Anzeige (2), einem Speicher, einer Steuerelektronik, einem D/A-Wandler, einer Spule (6) zum Applizieren von Magnetfeldern, einem Laser (7) zum Applizieren von Licht, sowie einer Sende- und Empfangseinrichtung zum Abfragen von Frequenzinformationen von einem Server, wobei die Vorrichtungen zumindest einen Sensor (5) zur Messung des Pulswertes umfasst, **dadurch gekennzeichnet, dass** die Vorrichtung ein Mobiltelefon (1) ist, die Frequenzinformationen in Form eines digitalen Dateiformats vorliegen, im Speicher des Mobiltelefons (1) ablegbar und über den D/A-Wandler auf die Spule (6) und den Laser (7) übertragbar sind, wobei das Mobiltelefon (1) zumindest zwei Sensoren (4) zur Messung des elektrischen Potentials des Herzens umfasst die Spule (6) ein Magnetfeld mit einer fluktuierenden Frequenz mit einem Mittelwert von 9 Hz und einer Magnetfeldstärke von 0,1 - 70 µT bei einer Messentfernung von 0 Metern erzeugt, und der Laser (7) eine Halbleiterlaserdiode ist, welche Licht mit einer fluktuierenden roten Wellenlänge mit einem Mittelwert von 650 nm erzeugt, und eine Leistung von weniger als 1 mW, bevorzugt etwa 0,5 mW bei etwa 15 mW/cm² aufweist.

2. Vorrichtung nach Anspruch 1, **dadurch gekennzeichnet, dass** das Magnetfeld symmetrisch quer zur Längsachse der Vorrichtung ausgerichtet ist.

3. Vorrichtung nach Anspruch 1 oder 2, **dadurch gekennzeichnet, dass** die Vorrichtung drei Sensoren (4) zur Messung des elektrischen Potentials des Herzens sowie einen Sensor (5) in Form eines Phototransistors zur Messung des Pulswertes umfasst.

4. Vorrichtung nach Anspruch 3, **dadurch gekennzeichnet, dass** drei Metallsensoren (4) in Form von Elektroden aus beispielsweise Ag/AgCl beschichteten Kunststoffkörpern und ein Photosensor (5) jeweils im Randbereich der Vorrichtung zur Auflage von jeweils einem Finger pro Sensor angeordnet sind.

5. Vorrichtung nach Anspruch 3 oder 4, **dadurch gekennzeichnet, dass** die Steuerelektronik der Vorrichtung die von den Sensoren empfangenen Signale zur Berechnung einer Herzratenvariabilitäts-Herzkohärenz-Analyse verarbeitet und das Ergebnis auf der Anzeige (2) der Vorrichtung ausgibt.

6. Vorrichtung nach Anspruch 5, **dadurch gekennzeichnet, dass** das von der Steuerelektronik errechnete Ergebnis mit einer im Speicher der Vorrichtung befindlichen Datenbank verglichen wird und an der Anzeige (2) der Vorrichtung eine entsprechende Auswahl für applizierbare Frequenzinformationen sowie zusätzliche weitere Informationen angezeigt werden können, wobei die angezeigte Auswahl an applizierbaren Frequenzinformationen mittels der Sende- und Empfangseinrichtung von einem Server herunterladbar sind

7. Vorrichtung nach einem der Ansprüche 3 bis 6, **dadurch gekennzeichnet, dass** die Sensoren (4, 5), der Speicher, die Steuerelektronik, der D/A-Wandler sowie die Spule (6) und/oder der Laser (7) in einem gemeinsamen Modul angeordnet sind, welches mit einem Mobiltelefon verbindbar oder in dieses einbaubar ist.

8. Vorrichtung nach Anspruch 7, **dadurch gekennzeichnet, dass** das Modul ferner eine eigene Stromversorgung, beispielsweise einen Akkumulator umfasst.

9. Vorrichtung nach einem der Ansprüche 7 oder 8, **dadurch gekennzeichnet, dass** das Modul mit dem Mobiltelefon (1) mittels einer Schnittstelle, wie etwa einer Bluetooth und/oder einer USB-Schnittstelle verbindbar ist.

## Claims

1. Device for using saved frequency information on the body, having a display (2), a memory, electronic controls, a D/A converter, a coil (6) for applying magnetic fields, a laser (7) for applying light, and a transmitter/receiver for interrogating frequency information from a server, the device comprising at least one sensor (5) for measuring the pulse, **characterised in that** the device is a mobile telephone (1), the frequency information is present in the form of a digital file format, can be saved in the memory of the mobile telephone (1) and can be transferred through the D/A converter to the coil (6) and the laser (7), the said mobile telephone (1) comprising at least two sensors (4) for measuring the electric potential of the heart, the coil (6) generating a magnetic field with a fluctuating frequency with an average value of 9 Hz and a magnetic field strength of 0.1 to 70 µT at a measuring distance of 0 metres, and the laser (7) being a semiconductor laser diode that generates light with a fluctuating red wavelength with an average value of 650 nm, and having an output of less than 1 mW, preferably about 0.5 mW at about 15 mW/cm².

2. Device according to claim 1, **characterised in that** the magnetic field is aligned symmetrically transversely to the longitudinal axis of the device.

3. Device according to claim 1 or 2, **characterised in that** the device comprises three sensors (4) for measuring the electrical potential of the heart and a sensor (5) in the form of a phototransistor for measuring the pulse.

4. Device according to claim 3, **characterised in that** three metal sensors (4) in the form of electrodes comprising for example Ag/AgCl coated plastics bodies and a photosensor (5) are disposed in the edge region of the device, for the application of one finger to each sensor.

5. Device according to claim 3 or 4, **characterised in that** the electronic controls of the device process the signals received from the sensors for the purpose of calculating a heart rate variability / heart coherence analysis and delivers the result on the display (2) of the device.

6. Device according to claim 5, **characterised in that** the result calculated by the electronic controls is compared with a database located in the memory of the device, and a corresponding selection of applicable frequency information and additional further information can be displayed on the display (2) of the device, the selection of applicable frequency information displayed being downloadable from a server by means of the transmitter/receiver.

7. Device according to one of claims 3 to 6, **characterised in that** the sensors (4, 5), the memory, the electronic controls, the D/A converter and the coil (6) and/or the laser (7) are disposed in a shared module which can be connected to a mobile telephone or can be incorporated therein.

8. Device according to claim 7, **characterised in that** the module further comprises its own power supply, for example a battery.

9. Device according to one of claims 7 or 8, **characterised in that** the module can be connected to the mobile telephone (1) by means of an interface such as, for example, a Bluetooth and/or a USB interface.

## Revendications

1. Dispositif pour appliquer sur le corps des informations de fréquence mémorisées, comportant un moyen d'affichage (2), une mémoire, une électronique de commande, un convertisseur D/A, une bobine (6) pour l'application de champs magnétiques, un laser (7) pour l'application de lumière, ainsi qu'un dispositif d'émission et de réception pour appeler des informations de fréquence à partir d'un serveur, le dispositif comprenant au moins un capteur (5) de mesure de la valeur de pulsation, **caractérisé par le fait que** le dispositif est un téléphone mobile (1), que les informations de fréquence se présentent sous la forme d'un format de données numériques, peuvent être enregistrées dans la mémoire du téléphone mobile (1) et transmises par l'intermédiaire du convertisseur D/A à la bobine (6) et au laser (7), le téléphone mobile (1) comportant au moins deux capteurs (4) pour mesurer le potentiel électrique du coeur, que la bobine (6) produit un champ magnétique avec une fréquence fluctuante de valeur moyenne 9Hz et une intensité de champ magnétique de 0,1 - 70 µT mesurée à une distance de 0 mètres, et le laser (7) est une diode laser à semi-conducteur qui produit de la lumière avec une longueur d'onde fluctuante dans le rouge de valeur moyenne 650 nm et une puissance inférieure à 1 mW, de préférence d'environ 0,5 mW à environ 15 mW/cm².

2. Dispositif selon la revendication 1, **caractérisé par le fait que** le champ magnétique est orienté de manière symétrique transversalement à l'axe longitudinal du dispositif.

3. Dispositif selon la revendication 1 ou 2, **caractérisé par le fait que** le dispositif comporte trois capteurs métalliques (4) sous la forme d'électrodes pour mesurer le potentiel électrique du coeur ainsi qu'un capteur (5) sous la forme d'un phototransistor pour la mesure de la valeur de la pulsation.

4. Dispositif selon la revendication 3, **caractérisé par le fait que** trois capteurs métalliques (4) sous la forme d'électrodes, sous la forme de corps en matière plastique revêtue par exemple de Ag/AgCl et un capteur photoélectrique (5) sont disposés chaque fois dans la région du bord du dispositif de manière à placer chaque fois un doigt sur chacun des capteurs.

5. Dispositif selon la revendication 3 ou 4, **caractérisé par le fait que** l'électronique de commande du dispositif traite les signaux reçus des capteurs afin d'effectuer une analyse de cohérence cardiaque - variabilité de la fréquence cardiaque et délivre le résultat sur le moyen d'affichage (2).

6. Dispositif selon la revendication 5, **caractérisé par le fait que** le résultat calculé par l'électronique de commande est comparé avec une base de données stockée dans la mémoire du dispositif, et que, sur le moyen d'affichage (2) du dispositif, peut être affichée une sélection correspondante d'informations de fréquences applicables ainsi que d'autres informations complémentaires, la sélection affichée d'informations de fréquences applicables pouvant être téléchargée à partir d'un serveur au moyen du dispositif d'émission - réception.

7. Un dispositif selon l'une des revendications 3 à 6, **caractérisé par le fait que** les capteurs (4, 5), la mémoire, l'électronique de commande, le convertisseur D/A ainsi que la bobine (6) et/ou le(s) laser(s) (7) sont disposés dans un module commun qui peut être relié à un téléphone mobile ou intégré dans celui-ci.

8. Dispositif selon la revendication 7, **caractérisé par le fait que** le module comprend également une alimentation électrique propre, par exemple un accumulateur.

9. Dispositif selon l'une des revendications 7 ou 8, **caractérisé par le fait que** le module est relié au téléphone mobile (1) par le biais d'une interface, telle que par exemple une interface Bluetooth et/ou une interface USB.
